# EUROPEAN PATENT APPLICATION

(11) **EP 4 292 978 A1**
(43) Date of publication of application: **20.12.2023**
(21) Application number: 23176753.4
(22) Date of filing: 01.06.2023
(51) Int. Cl.: C01B 3/06, C01B 3/04, C01B 13/02, G01N 27/04, G01N 27/06, G01N 27/22

(54) **REACTOR FOR DISSOCIATION OF AQUEOUS SOLUTIONS AND METHOD OF DISSOCIATION OF AQUEOUS SOLUTIONS**

(30) Priority: 03.06.2022 CZ 20220237; 13.12.2022 WO PCT/CZ2022/050130
(71) Applicant: Adasune s.r.o., 50004 Hradec Kralove (CZ)
(72) Inventor: Korbel, Viktor, 37802 Simanov (CZ)
(74) Representative: Sedlák, Jirí

(57) **Abstract**

The invention relates to a reactor (1) and to a method of using it, which comprises a hermetically sealed container (2) inside which are electrodes (3). After application of an aqueous solution between the electrodes (3), it is possible by controlled charging of the electrodes (3) between them to create an intense non-uniform electric field which begins to vibrate the molecules of the aqueous solution sufficiently to cause dissociation of the aqueous solution into dissociation products.

## Description

### Field of technology

The inventive device and method are used to dissociate aqueous solution molecules using an electric field. The products of the dissociation of aqueous solutions can serve as fuel in the energy industry or be subjected to measurements to analyse the composition of the aqueous solution.

### State of the art

The products of the dissociation of aqueous solutions can be used as fuel, as oxygen and hydrogen are among the products of the dissociation of aqueous solutions. It is a well-known fact that ways are currently being sought to address the energy crisis caused by the exclusion of fossil fuels from the energy industry. And one of the possible substitutes for fossil fuels is hydrogen gas, which appears to be a suitable candidate for replacing the burning of fossil fuels.

Hydrogen gas has the advantage of being widespread in the environment in the form of water. Each molecule of water contains two hydrogen atoms and one oxygen atom. When hydrogen burns, a large amount of energy is released and the product of combustion with oxygen is water vapour. This is a big advantage over fossil fuels, which release greenhouse gases, toxins and dust when they burn.

The disadvantage of these pure gases is their high reactivity, which is very dangerous in case of improper handling, accident or malfunction. It is therefore desirable that the pure gases should not be stored in large volumes in tanks, but that there should be a facility and method for their continuous production before immediate consumption. This would be advantageous, since clean water would be stored in the tanks as a feedstock, the eventual leakage of which from the storage tank is a minor safety risk.

Another disadvantage of using pure hydrogen and oxygen as a replacement for fossil fuels is the complexity of production, as breaking down a water molecule into atoms is a relatively complex process.

An example of the well-known production of hydrogen from water molecules is electrolysis, in which water is mixed with an electrolyte and then a direct electric current is passed through it. At one of the electrodes, the electric current causes oxygen to be emitted from the electrolyte in addition to other atoms, and at the other electrode, hydrogen is emitted from the electrolyte in addition to other atoms. Adversely, during electrolysis, the other atoms bind to other atoms present, in particular to atoms of the electrode material, and coatings are formed which may act as an insulating material making further electrolysis difficult. Furthermore, it is disadvantageous to work with DC electric current, which is very life-threatening even at low voltage levels. Last but not least, it is disadvantageous that electrolyte and electrodes have to be used, which complicates the consideration of simply using water, as the only feedstock, in the hydrogen and oxygen production process.

At the same time, it is known that the cleavage products of aqueous solutions can be used in the analysis of the chemical composition of aqueous solutions. It is a fact that there is a growing demand among those skilled in the art for an analysis of the chemical composition of aqueous solutions which is rapid, reliable and at the same time not demanding on the level of expertise of the analyst.

Chemical compositional analysis is known in which the analyte, mixture or aqueous solution is exposed to known chemical agents with high reactive potential, in particular acids, alkalis, free radicals. These agents react with the unknown substance, mixture or aqueous solution to form known salts and chemical compounds, from the presence of which the composition of the analyte is subsequently determined.

Chemical analysis requires a laboratory and a worker with a high level of expertise. In addition, performing chemical analysis is dangerous and time-consuming.

Another well-known method of compositional analysis is spectrometry, which uses electromagnetic radiation. The atoms of each element present in the sample being analysed absorb electromagnetic radiation, or emit electromagnetic radiation, of a specific wavelength spectrum. By knowing this behaviour of the atoms, it is possible to determine the chemical composition of the sample being analysed.

Spectrometric methods are very accurate but require very well calibrated instruments, including properly prepared samples. On the other hand, optical spectrometric instruments can be operated by trained personnel without a chemistry background. The disadvantage of spectrometric analysis is that it cannot analyse the composition in the optically opaque sample to be analysed.

This problem of an optically opaque sample is solved by spectrometric methods using ionising radiation, e.g., fluorescence analysis, where secondary radiation is detected on an ionising radiation detector. The disadvantage of these methods is that ionising radiation is a health hazard.

Another well-known type of compositional analysis is thermal compositional analysis, in which the sample is exposed to increasing temperatures. By knowing the behaviour of the chemical substances, it is possible to determine the composition of the analysed sample by the temperature at which changes occur in the sample (evaporation, chemical reactions with the atmosphere, etc.).

Even this method of composition analysis is not very suitable for mass deployment. Firstly, it requires a controlled environment with the possibility of controlled heating, including the capture of products released with increasing temperature of the sample being analysed. In addition, this analysis must be carried out by a trained person.

The object of the invention is to create a device for dissociation aqueous solutions, which could only be operated by a trained worker, which could split aqueous solutions to such an extent that the products of the dissociation could be used as a substitute for fossil fuels, Alternatively, it may be possible to determine the composition of the aqueous solution from the electrical characteristics of the fission process, or to introduce the fission products into a gas analyser to analyse the composition of aqueous solutions with a detector designed for a completely different state of matter. At the same time, it is an object of the invention to provide a method for cleaving aqueous solutions that is energy sustainable, without additional feedstock, and that is rapid for producing cleavage products in abundance.

### Summary of the invention

The problem is solved by using a reactor for dissociation aqueous solutions according to the invention below.

The essence of the invention is that the reactor comprises a hermetically sealed container made of an electrically inert material, and further comprising at least two electrodes inverted to each other, which are inserted into the hermetically sealed container of the reactor. This arrangement yields a hermetically and electrically sealed electrode capacitor whose dielectric will be an aqueous solution. At the same time, it is important in the context of the invention that the reactor exhibits at least one light source for irradiating the space inside the reactor with photons. The cleavage products of the aqueous solution are highly reactive with a tendency to form new chemical bonds. By supplying energy with photons, their tendency to form chemical bonds is partially suppressed and the fission products can therefore be diverted for further processing. At the same time, the reactor is provided with at least one opening for venting the gaseous fission products.

The advantages include that the reactor construction is simple but efficient. The electrodes define the gap(s) for flooding with the aqueous solution. The reactor is safe and can be sized as required.

Preferably, the reactor is provided with at least one aqueous solution supply for continuous replenishment of the aqueous solution and uninterrupted operation of the dissociation of the aqueous solution in the reactor.

It is also advantageous if the light source comprises a light emitting diode, preferably a UV light emitting diode. Light emitting diodes produce less waste heat than conventional filament light sources. In addition, they are suitable for miniaturisation, and are durable. UV light-emitting diodes produce light at a wavelength that is shown in current experiments to be the most optimal for stabilizing fission product ions.

In some cases, magnetic fields are used to reduce the tendency of fission products to form chemical bonds, so it is advantageous if the reactor is equipped with at least one source of electromagnetic radiation to stabilize the ions in the reactor space. The fission products gain energy from the incident photons, thereby reducing their reactivity, which would otherwise cause a high rate of recombination into new molecules. Similarly, the magnetic field source helps to stabilize the fission product ions by arranging their spatial orientation within the sample through magnetic interaction between the magnetic field and the magnetic dipole of the fission products.

Preferably, a gas analyser is included behind the reactor opening for analysing the composition of the fission products. Data is obtained from the gas analyser to refine the results of the analysis by comparing the electrical characteristics of the cleavage process of an aqueous solution of unknown composition with empirically obtained cleavage results of known aqueous solutions.

It is advantageous if the electrodes placed in the reactor are in the form of concentrically arranged and interposed cylinders. Experiments have shown that the behaviour of the electric field between cylindrical electrodes embedded in each other has a positive effect on the dissociation of molecular bonds.

It is further preferred if the electrodes are plate-shaped, wherein they are provided with a means for changing the width of the gap between the electrodes. By changing the electrode spacing, the thickness of the dielectric lying between the electrodes is substantially changed, which affects the electric field generated between the electrodes. If the distance between the electrodes is varied during the periodic generation of a non-uniformly increasing electric field, an additional variable is introduced into the aqueous solution system, thereby reducing the probability that the aqueous solution molecules will assume an equilibrium state and will not build up the energy required to dissociate their molecular bonds.

The invention also includes a method for dissociation aqueous solutions in a reactor according to the invention.

The essence of the invented method of dissociation aqueous solutions in a reactor is that a) the aqueous solution is poured between electrodes, where it takes the role of the dielectric of the capacitor. The aqueous solution as dielectric is exposed to an electric field when the electrodes are charged.

Further, the essence of the inventive method is that (b) a nonlinearly increasing electric field is generated between the electrodes by including the reactor electrodes in a series-connected tuned LC circuit, until some molecules of the aqueous solution are decomposed into fission products, wherein the ions of the fission products of the aqueous solution are stabilized by irradiation with photons or a magnetic field. A variable and increasingly intense electric field will excite the aqueous solution molecules so intensely that they break down to form fission products.

Further, the essence of the inventive method is that, after c), the fission products are discharged through an opening outside the reactor, whereupon method steps b) and c) are repeated as long as the aqueous solution is present between the electrodes. At the same time, in method step (b), the parameters of the non-uniformly increasing electric field are adjusted according to the current electrical capacitance of the capacitor formed by the electrodes and the current amount of aqueous solution, while the frequency of charging of the electrodes is varied from 50 kHz to 1 MHz. As the aqueous solution is converted to fission products, the electrical properties of the electrodes and the dielectric (capacitor) change, so the applied electric field gradually adapts to this change. It is also important that as the electrical conductivity of the aqueous solution increases, the frequency of charging of the electrical charge in the capacitor increases. The fission products act as charge carriers, so that the conductivity of the dielectric between the electrodes increases with increasing concentration. It is necessary to respond to this by changing the charging frequency.

Among the advantages of the inventive method is that the molecules of the aqueous solution are electrically decomposed without the need for the input of additional raw materials. The decomposition of aqueous solution molecules by means of an electric field is a cheap, efficient and almost maintenance-free method, which cannot be said, for example, of methods based on electrolysis, which need massive electrodes.

In a preferred method according to the invention, during method step (b), the electrical charging and discharging characteristics of the electrodes with the aqueous solution dielectric are measured, and the electrical charging and discharging characteristics of the electrodes with the aqueous solution are compared with an empirically obtained database of electrical charging and discharging characteristics of electrodes with aqueous solutions of known composition to determine the composition of the currently grafted aqueous solution.

Experiments show that the electrical characteristics are unique for different compositions of aqueous solutions, so it is possible to analyse the composition of currently unknown aqueous solutions by comparing the currently measured characteristics with archived characteristics of aqueous solutions of known composition.

### Description of drawings

The said invention will be explained in more detail in the following illustrations, where:
- Figure 1: shows in a simplified graphic a periodically recurring increase in the electrical voltage applied to the reactor electrodes,
- Figure 2: shows a simplified reactor model of the device,
- Figure 3: shows the bottom view of the reactor model in Figure 2.

### Example of the embodiment of invention

It is understood that the specific embodiments of the invention described and illustrated below are presented for purposes of illustration and not as a limitation of the invention to the examples provided. Those skilled in the art will find or be able to provide, using routine experimentation, a greater or lesser number of equivalents to the specific embodiments of the invention described herein.

The dissociation of aqueous solution molecules is achieved by repeatedly exposing the aqueous solution molecules to an extreme electric field of varying tendency that can supply the molecule atoms with the necessary excitation energy to overcome their mutual bonding.

The electric field must increase nonlinearly in its value in order for the molecules to be energetically stressed by the increase and decrease of the field. In macroscopic layman's terms, to more easily visualize the effect, the molecules of the aqueous solution must be vibrated with increasing intensity until the atoms bounce off each other.

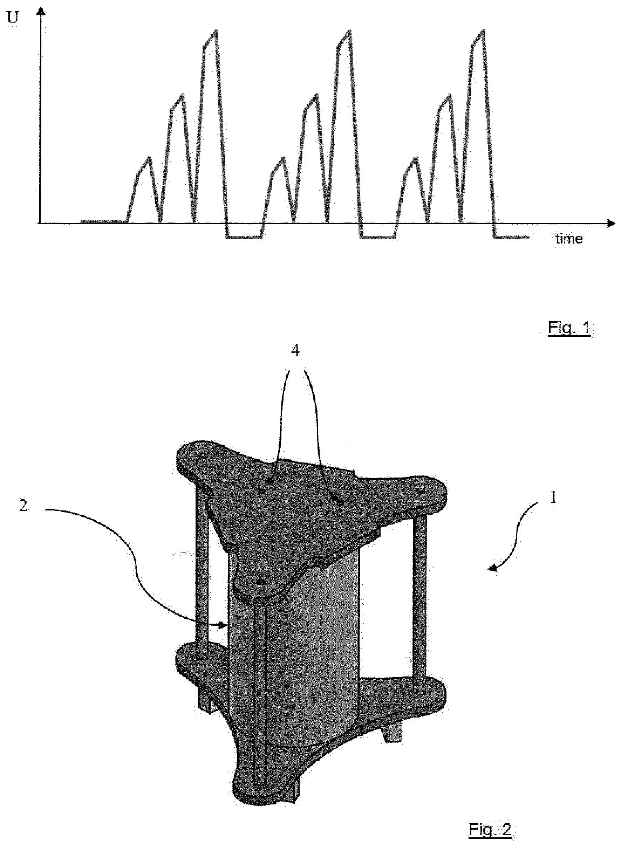

The electric field is generated between the electrodes 3 of the so-called capacitor, the dielectric of which is the aqueous solution. The non-linear increase in the electric voltage, which directly proportional to the electric charge on electrodes 3, is shown in Figure 1. As can be seen from the figure, the electric voltage reaches a certain limit, then decreases, then reaches a slightly higher limit, and this repeats until the molecules of the aqueous solution release electrons during their dissociation, which momentarily causes the electric voltage to drop below the horizontal axis of Figure 1. Charging takes place only in the positive half-plane.

This process is repeated continuously as long as aqueous solution is present between the electrodes 2 of the capacitor and as long as fission products need to be produced.

The frequency of the rise and fall of the electrical voltage is modified in Figure 1 to help understand the method, but in reality, the operating frequency ranges from 50 kHz to 1 MHz, which means up to 1 000 000 repetitions of the phenomenon in one second. This extreme stress on the bonds in the aqueous solution molecules causes them to break down. In addition, it has been verified that the conductivity of the aqueous solution between the electrodes decreases with increasing frequency. This is important as it is possible to process any aqueous solution. Due to the higher frequencies, the electrical charge is concentrated at electrodes 3 and an inactive region is formed in the gap between electrodes 3 in terms of electrical conductivity. Practical experiments with the laboratory reactor 1 showed that frequencies up to 280 kHz were sufficient for distilled water, frequencies from 720 kHz to 1 MHz were applicable for salted water with 5 % salinity table salt, while frequencies from 430 kHz to 650 kHz worked for tap water.

As for the amplitudes of the working electrical voltage, this depends on several factors. Firstly, it depends on the electrical properties of the aqueous solution to be treated, then on the surface area of the electrodes 3 and the distance of the electrode surfaces 3 from each other, then on the amount and temperature of the water, then on the degree of dissociation of the molecules, etc.

For this reason, the electrodes 3 of reactor 1 are connected to a tuned LC circuit which, through tuning, responds to the instantaneous demand of the electrical system to generate the desired extreme electric field. The advantage of the LC circuit is that when it goes into resonance, it can discharge and charge the electric charge in the electrodes 3 with the desired frequency, and the magnitude of the electric charge can be increased according to the tendency depicted in FIG. 1. The power source of the LC circuit is so-called feedback, which means that it responds to changes by itself. A feedback coil is used in the source, which when charged, closes the source tube, causing an oscillation - frequency. The use of a controlled power supply cannot be ruled out, but such a solution appears at first sight to be unnecessarily complex.

The aqueous solution may be gradually reduced and the power supply responds automatically, or the aqueous solution may be continuously replenished, which is again handled by the power supply for the electrodes 3 according to the above principle.

Since the dissociation of the aqueous solution molecules releases an electric charge in the form of electrons that are drawn to one of the electrodes 3, it is necessary to stabilize the product ions to prevent recombination into other new molecules. The electric charge is supplied to the fission product ions by irradiation with photons, ideally UV light, whose photons have sufficient energy to stabilize them, and all that has to happen is that the fission product ion is struck by the UV photon. It is also possible to use photons of light from the visible spectrum, or energetic particles from sources other than light.

In order to reduce the probability of molecular reconnection, in addition to bombardment by photons, the fission products are spatially arranged in the sample volume, which is achieved by applying an artificial magnetic field to their magnetic dipole. The means for the magnetic action may be a permanent magnet, such as a neodymium magnet, or a coil for emitting an induced magnetic field.

### Example 1

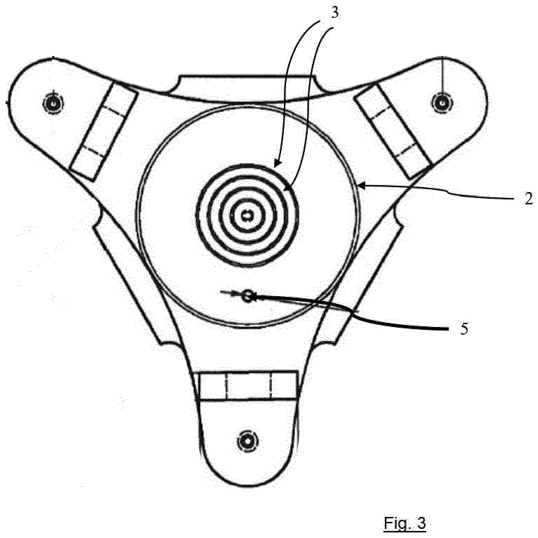

Figure 2 shows reactor 1 for dissociation aqueous solutions. The reactor 1 exhibits a hermetically sealable Plexiglas container 2, which is cylindrical in shape and closed by two flanges. Inside the hermetically sealed container 2 there are electrodes 3 of tubular shape, which are concentrically arranged and spaced apart for flooding with water. There are five tubular electrodes 3 in total, as shown in Figure 3.

Figures 2 and 3 also show openings 4 for the removal of gaseous fission products, and opening 5 for the continuous supply of aqueous solution.

In the non-depicted example embodiment of the invention, the electrodes are planar and are parallel to each other to create a free space between them for the dielectric, which will be the sample of the aqueous solution to be analysed. The electrodes are of high-quality stainless steel to resist wear and tear from the electric field and reactive ions of the fission products. The distance between the electrodes is adjustable. The distance adjustment is carried out by a plastic sleeve which is coupled to a plastic threaded rod which is rotated at one end by the transmission of mechanical force from a servomotor controlled by an external control unit or operator.

The high-frequency voltage source is principally built from a Tesla transformer, which works on the resonant principle. An important part of the power supply is an electronic high-frequency oscillator, from which the high-frequency voltage is applied to the electrodes of the capacitor, whereas in a conventional Tesla transformer the voltage is applied to a coil to generate secondary discharges, e.g., for testing the insulation strength of materials. The damped spark oscillator is adapted for read-back to respond to changes in the capacitance of the electrodes 3 with the aqueous solution of the reactor 1 and remain in resonance.

In addition to obtaining fission products for the energy and chemical industries, the invention can be used to analyse the composition of an unknown aqueous solution. Again, it takes advantage of the fact that an electric field which increases unevenly and repeats periodically acts on the molecules and atoms forming a sample of the aqueous solution to impart energy to them which, in critical amounts, leads to the breaking of molecular bonds to form ions from the molecules or to the ionization of the atoms. The addition of ions in the sample changes the electrical parameters of the electrode array 3 and its dielectric formed by the sample under test. These actual electrical parameters are compared with empirically collected values, thereby identifying the components of the unknown aqueous solution sample.

While the above methods of compositional analysis in the prior art use highly reactive chemicals (acids, alkalis), or high temperatures leading to decomposition of the sample for subsequent analysis of emissions, or dangerous ionizing radiation, the present invention can prepare ions of atoms originally forming the sample easily and safely with respect to the operator and the environment. Moreover, the invention can also be operated by a trained person who is only tasked with loading the sample into reactor 1, starting the automated analysis and allowing it to run to completion, whereupon the trained person only processes the results obtained.

The reactor 1 has an opening 4 in its lid for venting the gaseous fission products, to which a gas line can be fitted to the gas analyser. The gas analyser comprises a membrane gas and vapor detector or a semiconductor chip sensitive to condensates and gases. The gaseous matter analyser is a catalogue item that is purchased as a consumable item, so that the skilled person will be able to routinely apply the full range of available gaseous matter analysers to the invention.

### Example 2

A solution of water and NaCl and KCl salts was prepared. The salts were added to the water at a concentration of 8 % wt. The mixture was poured into the reactor as a sample. Subsequently, the frequency of the electric field between electrodes 3 was increased. The first changes in the electrical parameters were observed in the frequency region around 680 kHz, followed by frequencies around 700 kHz, 705 kHz, and 750 kHz. When compared with a database of empirically measured data, it was found that the changes detected at frequencies in the region 700 to 705 kHz correspond to water, the changes detected at frequencies in the region 680 kHz correspond to the presence of potassium ions, and the changes in the electrical parameters detected at frequencies in the region 750 kHz correspond to the presence of sodium ions.

At the same time, information on the presence of chlorine molecules in the water vapour was received from the gas analyser downstream of reactor 1 opening 4.

### Example 3

Wastewater from a septic tank containing solids was collected. The wastewater was mixed for 15 minutes in a blender. Subsequently, the mixture of water and solids was sieved through a 500 micrometre mesh sieve. Subsequently, the sieved effluent was poured into reactor 1 where it was exposed to an electric field. At the same time, the gaseous components were discharged to a gaseous analyser.

Changes in electrical parameters were measured at different frequencies, and for changes in the 150 kHz, 500 kHz, 700 kHz regions, records for the presence of nitrogen, carbon, water were found in the empirically collected data base.

The gas analyser detected the presence of hydrogen, oxygen, methane, chlorine and hydrogen sulphide.

The presence of these elements was verified by comparison with the results of laboratory analysis of the sample composition.

### Example 4

The sample from Example 3 was subjected to the electric fields in the same way as in Example 3, but with the difference that the distance between the electrodes 3 of reactor 1 was varied. The results of the measurements indicate that changing the distance (decreasing) between the electrodes 3 during the analysis has a positive effect on the dissociation of the molecules, since the gas analyser placed downstream of the opening 4 of reactor 1 detected gaseous components in higher concentration than in Example 3.

### Industrial applicability

The reactor for dissociation aqueous solutions and the method for dissociation aqueous solutions by the reactor according to the invention find application in the energy industry by burning the products of dissociation, in the chemical industry by processing the products of dissociation, or in analysing the composition of aqueous solutions of unknown composition.

## Claims

1. A reactor for dissociation aqueous solutions **characterized in that,** is comprising a hermetically sealed container (2) of electrically inert material, at least two electrodes (3) facing each other, which are inserted into the hermetically sealed container (2) of the reactor (1), at least one light source for irradiating the space inside the reactor (1) with photons, the reactor (1) having at least one opening (4) for the removal of gaseous fission products.

2. The reactor according to claim 1, **characterized in that** the reactor (1) is provided with at least one opening (5) for continuous supply of the aqueous solution.

3. The reactor of claim 1 or 2, **characterized in that** the light source comprises light emitting diodes.

4. The reactor according to claim 3, **characterized in that** the light source comprises a UV light emitting diode.

5. A reactor according to any one of claims 1 to 4, **characterized in that** the reactor (1) is provided with at least one source of electromagnetic radiation for stabilising ions in the reactor space (1).

6. The reactor according to any one of claims 1 to 5, **characterized in that** the reactor (1) comprises a gaseous analyser for analysing the composition of the fission products which is included downstream of the opening (4).

7. A reactor according to any one of claims 1 to 6, **characterized in that** the electrodes (3) are tubular in shape and are concentrically arranged in relation to each other.

8. The reactor according to any one of claims 1 to 6, **characterized in that** the electrodes (3) are plate-shaped and provided with means for changing the width of the gap between the electrodes (3).

9. A method of dissociation aqueous solutions by a reactor (1) according to any one of claims 1 to 8, **characterized in that** (a) the aqueous solution is poured between electrodes (3) where it takes the role of the dielectric of the capacitor, (b) a non-linearly increasing electric field is generated between the electrodes (3) by placing the electrodes (3) in a series-connected tuned LC circuit until some of the aqueous solution molecules decompose into fission products, the ions of the fission products of the aqueous solution being stabilized by photon irradiation or magnetic field, in step (c), the fission products are discharged through the opening (4) outside the reactor, whereupon method steps (b) and (c) are repeated for the time the aqueous solution is present between the electrodes (3), and in method step (b) the parameters of the non-uniformly increasing electric field are adjusted according to the current electric capacity of the capacitor formed by the electrodes (3) and the current amount of aqueous solution, while the charging frequency of the electrodes (3) is varied from 50 kHz to 1 MHz.

10. The method according to claim 9, **characterized in that** during method step b), the electrical characteristic of the charging and discharging of the electrodes (3) with the dielectric of the aqueous solution is measured, wherein the electrical charging and discharging characteristics of the electrodes (3) with aqueous solution are compared with an empirically obtained database of electrical charging and discharging characteristics of the electrodes (3) with aqueous solutions of known composition to determine the composition of the currently grafted aqueous solution.
